# FASCICULE DE BREVET EUROPEEN

(11) **EP 1 716 839 B1**
(45) Date de publication et mention de la délivrance du brevet: **19.09.2018**
(21) Numéro de dépôt: 06113224.7
(22) Date de dépôt: 27.04.2006
(51) Int. Cl.: A61K 8/22, A61K 8/92, A61Q 5/08, A61K 8/06

(54) **Emulsion directe comprenant une solution aqueuse de peroxyde d'hydrogène et une phase inerte de solubilité dans l'eau inférieure à 1 %**
Emulsion enthaltend eine wässrige Wasserstoffperoxidlösung und eine Phase mit einer wässrigen Löslichkeit niedriger als 1%
Emulsion comprising an aqueous solution of hydrogen peroxide and an inert phase having a solubility lower than 1%

(30) Priorité: 29.04.2005 FR 0551120
(43) Date de publication de la demande: 02.11.2006
(73) Titulaire: L'Oréal, 75008 Paris (FR)
(72) Inventeur: Kravtchenco, Sylvain, At Xintiandi,Lu Wan distr Shanghaï200021 (CN); Dubief, Claude, 78150, LE CHESNAY (FR)
(74) Mandataire: Prevel, Estelle Nicole

(56) Documents cités:
- EP-A- 1 291 006
- EP-A- 1 430 875
- WO-A-03/011216
- US-A1- 2004 235 700

## Description

La présente invention a pour objet une émulsion directe pour la décoloration des fibres kératiniques, et en particulier des fibres kératiniques humaines telles que les cheveux.

Lorsqu'une personne souhaite changer radicalement de couleur de cheveux, notamment lorsqu'elle souhaite obtenir une couleur plus claire que sa couleur d'origine, il est souvent nécessaire de procéder à une décoloration des cheveux. Pour ce faire, on utilise des produits de décoloration. Cette étape de décoloration est éventuellement associée à une étape de coloration des cheveux.

Il est connu de décolorer les fibres kératiniques, et en particulier les fibres kératiniques humaines telles que les cheveux, avec des compositions de décoloration contenant un ou plusieurs agents oxydants. Parmi les agents oxydants classiquement utilisés, on peut citer le peroxyde d'hydrogène, les composés susceptibles de produire le peroxyde d'hydrogène par hydrolyse tels que le peroxyde d'urée, ou les persels comme les perborates, les percarbonates et les persulfates, le peroxyde d'hydrogène et les persulfates étant particulièrement préférés.

Les compositions de décoloration se présentent principalement sous forme de produits anhydres, poudres ou crèmes, contenant des composés alcalins tels que les amines ou les silicates alcalins, et un réactif peroxygéné tels que les persulfates, les perborates ou les percarbonates, d'ammonium ou de métaux alcalins, que l'on dilue au moment de l'emploi avec une composition aqueuse de peroxyde d'hydrogène.

Les compositions de décoloration peuvent aussi résulter du mélange, au moment de l'emploi, d'une poudre anhydre contenant le réactif peroxygéné avec une composition aqueuse contenant les composés alcalins et une autre composition aqueuse contenant le peroxyde d'hydrogène.

Pour obtenir un produit de décoloration des fibres kératiniques qui soit plus efficace en terme d'éclaircissement et / ou de rapidité, il est en théorie possible d'augmenter la concentration en peroxyde d'hydrogène dans la composition aqueuse de peroxyde d'hydrogène. Cependant, une forte concentration en peroxyde d'hydrogène peut entraîner une dégradation des fibres kératiniques et éventuellement une irritation de la peau. De façon conventionnelle, dans des compositions classiques de décoloration, la concentration en peroxyde d'hydrogène est donc limitée à 12 % en poids, voire à 6 % en poids.

Par ailleurs, la demande de brevet EP 0 193 471 propose de décolorer les cheveux avec une solution anhydre de peroxyde d'hydrogène dans un solvant organique.

Il a aussi été proposé, dans la demande de brevet WO 03/011216, une composition pour la décoloration des cheveux qui se présente sous forme d'une émulsion directe ou inverse renfermant une solution aqueuse de peroxyde d'hydrogène et une phase organique comprenant au moins un composé perfluoré.

Toutefois, les résultats obtenus en terme d'éclaircissement ne sont pas encore satisfaisants.

Le but de la présente invention est de fournir de nouveaux produits d'éclaircissement qui soient plus efficaces que les produits d'éclaircissement connus de l'art antérieur tout en limitant la dégradation des fibres kératiniques et l'irritation de la peau.

Ce but est atteint avec la présente invention qui a pour objet une émulsion directe pour la décoloration des fibres kératiniques comprenant une phase inerte comprenant au moins un composé liquide non oxygéné et non perfluoré présentant une solubilité dans l'eau à 25 °C inférieure à 1 % et une solution aqueuse de peroxyde d'hydrogène, la phase inerte représentant au moins 20 % en poids du poids total de l'émulsion directe.

L'émulsion directe conforme à l'invention permet d'obtenir rapidement un éclaircissement important des fibres kératiniques tout en limitant la dégradation des fibres kératiniques et l'irritation de la peau.

La présente invention a aussi pour objet une composition de décoloration prête à l'emploi résultant du mélange de l'émulsion directe conforme à l'invention avec une ou plusieurs compositions comprenant chacune au moins un composé choisi parmi un agent alcalin, un persel, un colorant direct, un précurseur de colorant d'oxydation.

La présente invention a également pour objet un procédé de décoloration des fibres kératiniques mettant en oeuvre l'émulsion directe ou la composition de décoloration prête à l'emploi conformes à l'invention, ainsi qu'un kit pour la mise en oeuvre de ce procédé.

Un autre objet de l'invention est l'utilisation de l'émulsion directe ou de la composition de décoloration prête à l'emploi conformes à l'invention pour la décoloration des fibres kératiniques.

Dans le cadre de l'invention, on entend par émulsion directe une émulsion à phase aqueuse continue.

Dans le cadre de la présente invention, on entend par phase inerte une phase qui est chimiquement inerte vis-à-vis du peroxyde d'hydrogène. Dans le cadre de l'invention, une phase est inerte si la dégradation du peroxyde d'hydrogène en présence de cette phase est inférieure à 25 % après 15 heures à 100 °C.

Dans le cadre de la présente invention, on entend par composé liquide non oxygéné et non perfluoré tout dérivé organique ou minéral non oxygéné et non perfluoré pouvant s'écouler et possédant une viscosité à 25 °C inférieure à 5 000 Poises à un taux de cisaillement de 1 s⁻¹. Cette viscosité peut être déterminée à l'aide d'un viscosimètre de type RHEOMAT 180.

Selon un mode de réalisation particulier de l'invention, le ou les composés liquides non oxygénés et non perfluorés sont choisis parmi les polyalphaoléfines, les polybutènes et les polyisobutènes, les huiles minérales, les huiles de paraffines, les copolymères à blocs polystyrène et polyisoprène tels que les Kratons liquides, et leurs mélanges.

Selon un mode de réalisation particulier, la phase inerte représente entre 20 et 95 % en poids, et de préférence entre 30 et 90 % en poids du poids total de l'émulsion directe.

L'émulsion directe conforme à l'invention présente généralement une concentration en peroxyde d'hydrogène comprise entre 1 et 20 % en poids, de préférence entre 2 et 12 % en poids du poids total de l'émulsion directe.

Selon un mode de réalisation particulier, l'émulsion directe de l'invention comprend au moins un composé choisi parmi les agents tensioactifs anioniques, non ioniques, cationiques et amphotères, possédant une ou plusieurs chaînes alkylées en C₆-C₂₂ linéaires ou ramifiées et / ou une ou plusieurs chaînes perfluorées, les polymères amphiphiles tels que les copolymères à blocs amphiphiles hydrosolubles ou hydrodispersibles, les polymères associatifs porteurs sur leur chaîne principale de groupements alkyle et / ou aryle en C₆-C₂₂, oxyéthylénés ou non, téléchéliques ou greffés, ces polymères pouvant être de charge cationique, anionique, amphotère ou non ionique, et les polymères épaississants tels que les polymères acryliques réticulés, les polysaccharides naturels ou modifiés.

Ces composés peuvent être chacun présents dans l'émulsion directe conforme à l'invention en quantité comprise entre 0,1 et 30 % en poids, et de préférence entre 0,2 et 15 % en poids du poids total de l'émulsion directe.

L'émulsion directe conforme à l'invention présente un pH inférieur à 5. Le pH peut être ajusté à la valeur désirée au moyen d'agents acides ou alcalins habituellement utilisés en cosmétique ou bien encore à l'aide de systèmes tampons classiques.

Parmi les agents acides, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalins on peut citer, à titre d'exemple, l'ammoniaque, les carbonates alcalins, les alcanolamines telles que les di- et triéthanolamines ainsi que leurs dérivés, les hydroxydes de sodium ou de potassium et les composés de formule (III) suivante : 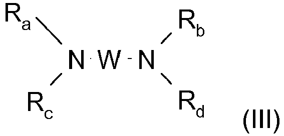 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Afin de décolorer les fibres kératiniques, l'émulsion directe conforme à l'invention peut être appliquée telle quelle sur les fibres kératiniques. Elle peut aussi être mélangée au moment de l'emploi avec une ou plusieurs compositions dans lesquelles sont répartis au moins un agent alcalin et / ou au moins un persel et / ou au moins un colorant direct et / ou au moins un précurseur de colorant d'oxydation, pour conduire à une composition de décoloration prête à l'emploi.

Dans le cas où la composition de décoloration prête à l'emploi comprend au moins un colorant direct et / ou au moins un précurseur de colorant d'oxydation, on réalise une coloration éclaircissante, c'est-à-dire que l'on obtient simultanément une décoloration et une coloration des fibres kératiniques.

Le ou les agents alcalins peuvent être choisis parmi les amines organiques, l'ammoniaque et les silicates.

Lorsque la composition de décoloration prête à l'emploi conforme à l'invention comprend un ou plusieurs agents alcalins, ils sont généralement présents en quantité comprise entre 0,01 et 40 % en poids, et de préférence entre 0,1 et 30 % en poids du poids total de la composition prête à l'emploi.

Le ou les persels peuvent être choisis parmi les perborates, les percarbonates et les persulfates, d'ammonium ou de métaux alcalins.

Lorsque la composition de décoloration prête à l'emploi conforme à l'invention comprend un ou plusieurs persels, ils sont généralement présents en quantité comprise entre 10 et 70 % en poids, et de préférence entre 20 et 60 % en poids du poids total de la composition prête à l'emploi.

Le ou les colorants directs peuvent être choisis parmi les colorants directs classiquement utilisés en coloration directe. A titre d'exemples, ces colorants directs sont choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels. Ces colorants directs peuvent être de nature non ionique, anionique ou cationique.

Parmi les colorants directs benzéniques, on peut citer le 1,4-diamino-2-nitrobenzène, le 1-amino-2-nitro-4-(β-hydroxyéthylamino)-benzène, le 1-amino-2-nitro-4-bis(β-hydroxyéthyl)-aminobenzène, le 1,4-bis(β-hydroxyéthylamino)-2-nitro-benzène, le 1-β-hydroxyéthylamino-2-nitro-4-bis-(β-hydroxyéthylamino)-benzène, le 1-β-hydroxyéthylamino-2-nitro-4-amino-benzène, le 1-β-hydroxyéthylamino-2-nitro-4-(éthyl)(β-hydroxyéthyl)-aminobenzène, le 1-amino-3-méthyl-4-β-hydroxyéthylamino-6-nitro-benzène, le 1-amino-2-nitro-4-β-hydroxyéthylamino-5-chloro-benzène, le 1,2-diamino-4-nitro-benzène, le 1-amino-2-β-hydroxyéthylamino-5-nitro-benzène, le 1,2-bis-(β-hydroxyéthylamino)-4-nitro-benzène, le 1-amino-2-[tris-(hydroxyméthyl)-méthylamino]-5-nitrobenzène, le 1-hydroxy-2-amino-5-nitro-benzène, le 1-hydroxy-2-amino-4-nitro-benzène, le 1-hydroxy-3-nitro-4-amino-benzène, le 1-hydroxy-2-amino-4,6-dinitro-benzène, le 1-β-hydroxyéthyloxy-2-β-hydroxyéthylamino-5-nitro-benzène, le 1-méthoxy-2-β-hydroxyéthylamino-5-nitro-benzène, le 1-β-hydroxyéthyloxy-3-méthylamino-4-nitrobenzène, le 1-β,γ-dihydroxypropyloxy-3-méthylamino-4-nitro-benzène, le 1-β-hydroxyéthylamino-4-β,γ-dihydroxypropyloxy-2-nitro-benzène, le 1-β,γ-dihydroxypropylamino-4-trifluorométhyl-2-nitro-benzène, le 1-β-hydroxyéthylamino-4-trifluorométhyl-2-nitro-benzène, le 1-β-hydroxyéthylamino-3-méthyl-2-nitro-benzène, le 1-β-aminoéthylamino-5-méthoxy-2-nitro-benzène, le 1-hydroxy-2-chloro-6-éthylamino-4-nitrobenzène, le 1-hydroxy-2-chloro-6-amino-4-nitro-benzène, le 1-hydroxy-6-[bis-(β-hydroxyéthyl)-amino]-3-nitro-benzène, le 1-β-hydroxyéthylamino-2-nitro-benzène, le 1-hydroxy-4-β-hydroxyéthylamino-3-nitro-benzène.

Parmi les colorants directs azoïques, on peut citer les colorants azoïques cationiques décrits dans les demandes de brevets WO 95/15144, WO-95/01772, EP 0 714 954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369 et FR 2 844 269 dont le contenu fait partie intégrante de l'invention.

Parmi ces composés, on peut tout particulièrement citer le chlorure de 1,3-diméthyl-2-[[4-(diméthylamino)phényl]azo]-1H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants: la 1-N-méthylmorpholiniumpropylamino-4-hydroxyanthraquinone, la 1-aminopropylamino-4-méthylaminoanthraquinone, la 1-aminopropylaminoanthraquinone, la 5-β-hydroxyéthyl-1,4-diaminoanthraquinone, la 2-aminoéthylaminoanthraquinone, la 1,4-bis-(β,□γ-dihydroxypropylamino)-anthraquinone.

Parmi les colorants aziniques, on peut citer les composés suivants : Basic Blue 17, Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants : la 2-β-hydroxyéthlyamino-5-[bis-(β-4'-hydroxyéthyl)amino]anilino-1,4-benzoquinone, la 2-β-hydroxyéthylamino-5-(2'-méthoxy-4'-amino)anilino-1,4-benzoquinone, la 3-N(2'-Chloro-4'-hydroxy)phényl-acétylamino-6-méthoxy-1,4-benzoquinone imine, la 3-N(3'-Chloro-4'-méthylamino)phényl-uréido-6-méthyl-1,4-benzoquinone imine, la 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Lorsque la composition de décoloration prête à l'emploi conforme à l'invention comprend un ou plusieurs colorants directs, ils sont généralement présents en quantité comprise entre 0,001 et 20 % en poids environ du poids total de la composition prête à l'emploi, et encore plus préférentiellement entre 0,005 et 10 % en poids environ.

Les précurseurs de colorants d'oxydation peuvent être choisis parmi les bases d'oxydation et les coupleurs conventionnellement utilisés dans le domaine de la coloration.

A titre d'exemples de bases d'oxydation, on peut citer les para-phénylènediamines, les bases doubles, les para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bases doubles, on peut citer, à titre d'exemples, les bis-phénylalkylènediamines et les bis-para-aminophénols.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsque la composition de décoloration prête à l'emploi conforme à l'invention comprend une ou plusieurs bases d'oxydation, elles sont généralement présentes en quantité comprise entre 0,001 et 10 % en poids environ du poids total de la composition prête à l'emploi, de préférence entre 0,005 et 6 % en poids environ.

A titre d'exemples de coupleurs, on peut citer les méta-phénylènediamines, les méta-aminophénols, les méta-diphénols, les coupleurs naphtaléniques, les coupleurs hétérocycliques et leurs sels d'addition.

A titre d'exemples, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Lorsque la composition de décoloration prête à l'emploi conforme à l'invention comprend un ou plusieurs coupleurs, ils sont généralement présents en quantité comprise entre 0,001 et 10 % en poids environ du poids total de la composition prête à l'emploi, de préférence entre 0,005 et 6 % en poids environ.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

Selon un mode de réalisation particulier, la composition de décoloration prête à l'emploi conforme à l'invention comprend au moins un agent alcalin tel que défini précédemment. Elle peut alors résulter du mélange de l'émulsion directe conforme à l'invention avec une composition aqueuse comprenant, dans un milieu cosmétique approprié, le ou les agents alcalins.

Selon un autre mode de réalisation particulier, la composition de décoloration prête à l'emploi conforme à l'invention comprend au moins un agent alcalin et au moins un persel tels que définis précédemment. Elle peut alors résulter du mélange de l'émulsion directe conforme à l'invention avec une composition anhydre comprenant, dans un milieu cosmétique approprié, le ou les agents alcalins et le ou les persels. Elle peut aussi résulter du mélange de l'émulsion directe conforme à l'invention avec une composition aqueuse comprenant, dans un milieu cosmétique approprié, le ou les agents alcalins et une composition anhydre comprenant, dans un milieu cosmétique approprié, le ou les persels.

Selon un autre mode de réalisation particulier, la composition de décoloration prête à l'emploi conforme à l'invention comprend au moins un colorant direct tel que défini précédemment. Elle peut alors résulter du mélange de l'émulsion directe conforme à l'invention avec une composition aqueuse comprenant, dans un milieu cosmétique approprié, le ou les colorants directs.

Selon un autre mode de réalisation particulier, la composition de décoloration prête à l'emploi conforme à l'invention comprend au moins un agent alcalin et au moins un colorant direct et / ou au moins un précurseur de colorant d'oxydation tels que définis précédemment. Elle peut alors résulter du mélange de l'émulsion directe conforme à l'invention avec une composition aqueuse comprenant, dans un milieu cosmétique approprié, le ou les agents alcalins et le ou les colorants directs et / ou le ou les précurseurs de colorant d'oxydation. Elle peut également résulter du mélange de l'émulsion directe conforme à l'invention avec une composition aqueuse comprenant, dans un milieu cosmétique approprié, le ou les agents alcalins et une composition aqueuse comprenant, dans un milieu cosmétique approprié, le ou les colorants directs et / ou le ou les précurseurs de colorant d'oxydation.

Selon un autre mode de réalisation particulier, la composition de décoloration prête à l'emploi conforme à l'invention comprend au moins un agent alcalin, au moins un persel et au moins un colorant direct tels que définis précédemment. Elle peut alors résulter du mélange de l'émulsion directe conforme à l'invention avec une composition anhydre comprenant, dans un milieu cosmétique approprié, le ou les agents alcalins et le ou les persels et une composition aqueuse comprenant, dans un milieu cosmétique approprié, le ou les colorants directs. Elle peut aussi résulter du mélange de l'émulsion directe conforme à l'invention avec une composition aqueuse comprenant, dans un milieu cosmétique approprié, le ou les agents alcalins, une composition anhydre comprenant, dans un milieu cosmétique approprié, le ou les persels et une composition aqueuse comprenant, dans un milieu cosmétique approprié, le ou les colorants directs.

Les compositions comprenant le ou les persels sont anhydres. Elles peuvent également comprendre des additifs usuels dans le domaine, en particulier des polymères épaississants hydrosolubles, des charges telles que des argiles ou de la silice amorphe, des liants tels que la vinylpyrrolidone, des lubrifiants comme les stéarates de polyol ou les stéarates de métaux alcalins ou alcalino-terreux, ainsi que des agents de contrôle du dégagement d'oxygène tels que le carbonate ou l'oxyde de magnésium, des agents colorants ou des agents matifiants comme les oxydes de titane ou encore des agents tensioactifs anioniques, non ioniques, cationiques ou amphotères, des vitamines.

A titre illustratif, la teneur en additif(s) représentent 0,01 à 40 % en poids, de préférence de 0,1 à 30 % en poids par rapport au poids total des compositions.

Les compositions anhydres peuvent se présenter sous forme de poudre ou de pâte. Dans le cas où elles se présentent sous forme de pâte, elles comprennent de plus un liquide inerte organique choisi parmi les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales.

Les autres compositions sont de préférence aqueuses. Le milieu cosmétique approprié de ces compositions est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids par rapport au poids total de la composition, et plus préférentiellement encore entre 5 et 30 % en poids environ.

Ces compositions peuvent également renfermer divers adjuvants utilisés classiquement dans les compositions de décoloration des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus peuvent être présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à ces compositions ne soient pas, ou substantiellement pas, altérées par la ou les adjonctions envisagées.

Ces compositions peuvent se présenter sous des formes diverses, telles que sous forme de liquides, de crèmes, de gels, ou sous toute autre forme cosmétique appropriée.

Le pH de la composition de décoloration prête à l'emploi conforme à l'invention est compris entre 4 et 11. Il est préférentiellement alcalin, et encore plus préférentiellement compris entre 7 et 11.

Le procédé de décoloration conforme à la présente invention consiste à appliquer sur les fibres kératiniques une émulsion directe ou une composition de décoloration prête à l'emploi conformes à l'invention telles que définies précédemment.

La présente invention a également pour objet un kit pour la décoloration des fibres kératiniques contenant une émulsion directe conforme à l'invention et une ou plusieurs compositions dans lesquelles sont répartis au moins un agent alcalin et / ou au moins un persel et / ou au moins un précurseur de colorant et / ou au moins un colorant direct tels que définis précédemment.

Selon un mode de réalisation particulier de l'invention, le kit conforme à l'invention contient une émulsion directe conforme à l'invention et une composition aqueuse comprenant, dans un milieu cosmétique approprié, au moins un agent alcalin tel que défini précédemment.

Selon un autre mode de réalisation particulier de l'invention, le kit conforme à l'invention contient une émulsion directe conforme à l'invention, une composition anhydre comprenant, dans un milieu cosmétique approprié, au moins un persel tel que défini précédemment et une composition aqueuse comprenant, dans un milieu cosmétique approprié, au moins un agent alcalin tel que défini précédemment.

Selon un autre mode de réalisation particulier de l'invention, le kit conforme à l'invention contient une émulsion directe conforme à l'invention et une composition anhydre comprenant, dans un milieu cosmétique approprié, au moins un agent alcalin et au moins un persel tels que définis précédemment.

Selon un autre mode de réalisation particulier de l'invention, le kit conforme à l'invention contient une émulsion directe conforme à l'invention et une composition aqueuse comprenant, dans un milieu cosmétique approprié, au moins un colorant direct tel que défini précédemment.

Selon un autre mode de réalisation particulier de l'invention, le kit conforme à l'invention contient une émulsion directe conforme à l'invention, une composition aqueuse comprenant, dans un milieu cosmétique approprié, au moins un agent alcalin tel que défini précédemment et une composition aqueuse comprenant, dans un milieu cosmétique approprié, au moins un colorant direct et / ou au moins un précurseur de colorant d'oxydation tels que définis précédemment.

Selon un autre mode de réalisation particulier de l'invention, le kit conforme à l'invention contient une émulsion directe conforme à l'invention et une composition aqueuse comprenant, dans un milieu cosmétique approprié, au moins un agent alcalin et au moins un colorant direct et / ou au moins un précurseur de colorant d'oxydation tels que définis précédemment.

Selon un autre mode de réalisation particulier de l'invention, le kit conforme à l'invention contient une émulsion directe conforme à l'invention, une composition anhydre comprenant, dans un milieu cosmétique approprié, au moins un agent alcalin et au moins un persel tels que définis précédemment et une composition aqueuse comprenant, dans un milieu cosmétique approprié, au moins un colorant direct tel que défini précédemment.

Selon un autre mode de réalisation particulier de l'invention, le kit conforme à l'invention contient une émulsion directe conforme à l'invention, une composition anhydre comprenant, dans un milieu cosmétique approprié, au moins un persel tel que défini précédemment, une composition aqueuse comprenant, dans un milieu cosmétique approprié, au moins un agent alcalin tel que défini précédemment et une composition aqueuse comprenant, dans un milieu cosmétique approprié, au moins un colorant direct tel que défini précédemment.

La présente invention a aussi pour objet l'utilisation pour la décoloration des fibres kératiniques d'une émulsion directe ou d'une composition de décoloration prête à l'emploi conformes à l'invention telles que définies précédemment.

Les exemples qui suivent servent à illustrer l'invention sans toutefois présenter un caractère limitatif.

### EXEMPLE

On a préparé la composition oxydante 1 suivante :

| **COMPOSITION OXYDANTE 1** | |
|---|---|
| Huile de paraffine | 50 g |
| Lauryl sulfate | 2 g |
| Alcool stéarylique oxyéthyléné (100 OE) ou Steareth-100 | 1 g |
| Acide polyacrylique Carbopol 940 commercialisé par la société Novéon | 2 g |
| Peroxyde d'hydrogène | 6 g |
| Stannate de sodium, 6 H₂O | 0,04 g |
| Acide diéthylène triamine pentacétique | 0,015 g |
| Eau déminéralisée | qsp 100 g |

La composition oxydante 1 est mélangée au moment de l'emploi à une poudre décolorante contenant 50 % de persulfates, 24,1 % de silicates et 2,6 % de chlorure d'ammonium, avec un rapport poudre décolorante / composition oxydante égal à 1 / 1,5.

Le mélange obtenu est appliqué sur des mèches de cheveux naturels châtains de 2,5 g, à raison de 10 g de mélange pour 1 g de cheveux. Après un temps de pose de 35 minutes, les mèches sont rincées, lavées avec un shampooing standard, rincées à nouveau puis séchées.

Le reflet obtenu est un blond foncé cuivré intense.

## Revendications

1. Emulsion directe pour la décoloration des fibres kératiniques comprenant une phase inerte comprenant au moins un composé liquide non oxygéné et non perfluoré présentant une solubilité dans l'eau à 25 °C inférieure à 1 % et une solution aqueuse de peroxyde d'hydrogène, la phase inerte représentant au moins 20 % en poids du poids total de l'émulsion directe, ladite émulsion comprenant au moins un composé choisi parmi les polymères amphiphiles choisis parmi les copolymères à blocs amphiphiles hydrosolubles ou hydrodispersibles, les polymères associatifs porteurs sur leur chaîne principale de groupements alkyle et / ou aryle en C₆-C₂₂, oxyéthylénés ou non, téléchéliques ou greffés, ces polymères étant de charge non ionique, cationique ou amphotère, présent en quantité comprise entre 0,1 et 30 % en poids du poids total de l'émulsion directe.

2. Emulsion directe selon la revendication 1, dans laquelle le ou les composés liquides non oxygénés et non perfluorés sont choisis parmi les polyalphaoléfines, les polybutènes et les polyisobutènes, les huiles minérales, les huiles de paraffines, les copolymères à blocs polystyrène et polyisoprène, et leurs mélanges.

3. Emulsion directe selon la revendication 1 ou 2, dans laquelle la phase inerte représente entre 20 et 95 % en poids du poids total de l'émulsion directe.

4. Emulsion directe selon la revendication 3, dans laquelle la phase inerte représente entre 30 et 90 % en poids du poids total de l'émulsion directe.

5. Emulsion directe selon l'une quelconque des revendications précédentes, dans laquelle la concentration en peroxyde d'hydrogène est comprise entre 1 et 20 % en poids du poids total de l'émulsion directe.

6. Emulsion directe selon la revendication 5, dans laquelle la concentration en peroxyde d'hydrogène est comprise entre 2 et 12 % en poids du poids total de l'émulsion directe.

7. Emulsion directe selon l'une quelconque des revendications 1 à 7, dont le pH est inférieur à 5.

8. Procédé de décoloration des fibres kératiniques, **caractérisé en ce que** l'on applique sur les fibres kératiniques une émulsion directe telle que définie à l'une quelconque des revendications 1 à 7.

9. Kit pour la décoloration des fibres kératiniques contenant une émulsion directe telle que définie à l'une quelconque des revendications 1 à 7 et une ou plusieurs compositions dans lesquelles sont répartis au moins un agent alcalin et / ou au moins un persel et / ou au moins un précurseur de colorant et / ou au moins un colorant direct.

10. Utilisation pour la décoloration des fibres kératiniques d'une émulsion directe telle que définie à l'une quelconque des revendications 1 à 7.

## Patentansprüche

1. Direkte Emulsion zur Entfärbung von Keratinfasern, umfassend eine inerte Phase, umfassend mindestens eine flüssige nicht-oxygenierte und nichtperfluorierte Verbindung mit einer Löslichkeit in Wasser bei 25°C von weniger als 1 %, und eine wässrige Wasserstoffperoxidlösung, wobei die inerte Phase mindestens 20 Gew.-% des Gesamtgewichts der direkten Emulsion darstellt, wobei die Emulsion mindestens eine Verbindung umfasst, ausgewählt aus amphiphilen Polymeren, ausgewählt aus wasserlöslichen oder wasserdispergierbaren amphiphilen Blockcopolymeren, wobei die assoziativen Polymere, die an ihrer Hauptkette C₆-C₂₂-Alkyl- und/oder -Arylgruppen, oxyethyleniert oder nicht, telechelisch oder gepfropft, tragen, wobei die Polymere, die eine nicht-ionische, kationische oder amphotere Ladung aufweisen, in einer Menge zwischen 0,1 und 30 Gew.-% des Gesamtgewichts der direkten Emulsion vorliegen.

2. Direkte Emulsion nach Anspruch 1, wobei die flüssige(n) nicht-oxygenierte(n) und nicht-perfluorierte(n) Verbindung(en) ausgewählt ist (sind) aus Polyalphaolefinen, Polybutenen und Polyisobutenen, Mineralölen, Paraffinölen, Polystyrol- und Polyisopren-Blockcopolymeren und ihren Mischungen.

3. Direkte Emulsion nach Anspruch 1 oder 2, wobei die inerte Phase zwischen 20 und 95 Gew.-% des Gesamtgewichts der direkten Emulsion darstellt.

4. Direkte Emulsion nach Anspruch 3, wobei die inerte Phase zwischen 30 und 90 Gew.-% des Gesamtgewichts der direkten Emulsion darstellt.

5. Direkte Emulsion nach einem der vorhergehenden Ansprüche, wobei die Wasserstoffperoxidkonzentration zwischen 1 und 20 Gew.-% des Gesamtgewichts der direkten Emulsion beträgt.

6. Direkte Emulsion nach Anspruch 5, wobei die Wasserstoffperoxidkonzentration zwischen 2 und 12 Gew.-% des Gesamtgewichts der direkten Emulsion beträgt.

7. Direkte Emulsion nach einem der Ansprüche 1 bis 7, deren pH kleiner ist als 5.

8. Verfahren zur Entfärbung von Keratinfasern, **dadurch gekennzeichnet, dass** auf die Keratinfasern eine direkte Emulsion, wie in einem der Ansprüche 1 bis 7 definiert, aufgebracht wird.

9. Kit zur Entfärbung von Keratinfasern, welches eine direkte Emulsion, wie in einem der Ansprüche 1 bis 7 definiert, und eine oder mehrere Zusammensetzungen umfasst, in denen mindestens ein alkalisches Mittel und/oder mindestens ein Persalz und/oder mindestens ein Vorläufer des Färbemittels und/oder mindestens ein direktes Färbemittel verteilt sind.

10. Verwendung einer direkten Emulsion, wie in einem der Ansprüche 1 bis 7 definiert, zur Entfärbung von Keratinfasern.

## Claims

1. Direct emulsion for bleaching keratin fibres, comprising an inert phase comprising at least one non-oxygenated and non-perfluoro liquid compound with a solubility in water at 25°C of less than 1% and an aqueous hydrogen peroxide solution, the inert phase representing at least 20% by weight relative to the total weight of the direct emulsion, said emulsion comprising at least one compound chosen from amphiphilic polymers chosen from water-soluble or water-dispersible amphiphilic block copolymers, associative polymers bearing oxyethylenated or non-oxyethylenated, telechelic or grafted C₆-C₂₂ alkyl and/or aryl groups on their main chain, these polymers having a nonionic, cationic or amphoteric charge, present in an amount of between 0.1% and 30% by weight relative to the total weight of the direct emulsion.

2. Direct emulsion according to Claim 1, in which the non-oxygenated and non-perfluoro liquid compound(s) are chosen from poly-alpha-olefins, polybutenes and polyisobutenes, mineral oils, liquid paraffins, copolymers bearing polystyrene and polyisoprene blocks, and mixtures thereof.

3. Direct emulsion according to Claim 1 or 2, in which the inert phase represents between 20% and 95% by weight relative to the total weight of the direct emulsion.

4. Direct emulsion according to Claim 3, in which the inert phase represents between 30% and 90% by weight relative to the total weight of the direct emulsion.

5. Direct emulsion according to any one of the preceding claims, in which the hydrogen peroxide concentration is between 1% and 20% by weight relative to the total weight of the direct emulsion.

6. Direct emulsion according to Claim 5, in which the hydrogen peroxide concentration is between 2% and 12% by weight relative to the total weight of the direct emulsion.

7. Direct emulsion according to any one of Claims 1 to 7, the pH of which is less than 5.

8. Process for bleaching keratin fibres, **characterized in that** a direct emulsion as defined in any one of Claims 1 to 7 is applied to the keratin fibres.

9. Kit for bleaching keratin fibres, containing a direct emulsion as defined in any one of Claims 1 to 7 and one or more compositions in which are distributed at least one alkaline agent and/or at least one persalt and/or at least one dye precursor and/or at least one direct dye.

10. Use for bleaching keratin fibres of a direct emulsion as defined in any one of Claims 1 to 7.
